(19) Europäisches Patentamt
European Patent Office
Office européen des brevets

(11) Veröffentlichungsnummer: **0 163 179 A1**

(12) # EUROPÄISCHE PATENTANMELDUNG

(21) Anmeldenummer: 85105736.4

(22) Anmeldetag: 10.05.85

(51) Int. Cl.⁴: A 61 L 15/00
A 01 N 25/10, A 01 N 59/20

(30) Priorität: 18.05.84 DE 3418521

(43) Veröffentlichungstag der Anmeldung:
04.12.85 Patentblatt 85/49

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Sustmann, Scarlet, Dr.
Am Nachtigallenwäldchen
D-4060 Viersen(DE)

(54) **Menstrualtampon.**

(57) Ein Menstrualtampon besteht aus einer äußeren Umhüllung aus Cellulosefasermaterial und einem Kern aus saugfähigem, ganz oder teilweise bakteriostatisch ausgerüstetem Fasermaterial. Das Fasermaterial des Kerns besteht aus mit anionischen Gruppen, bevorzugt mit Carboxymethlygruppen modifizierten Cellulosefasern, an welche bakteriostatisch wirksame Kationen, bevorzugt Kupfer-II-Ionen gebunden sind. Bevorzugt wird ein Fasermaterial aus einer einheitlichen carboxymethylierten Viskose, mit einem Substitutionsgrad von 0,01 bis 0,3 und ausgerüstet mit 0,01 bis 2,0, bevorzugt 0,2 bis 0,8 Gew.-% Kupferionen verwendet. Es werden Verfahren zur Herstellung der Mentrualtampons beschrieben.

Abb.I

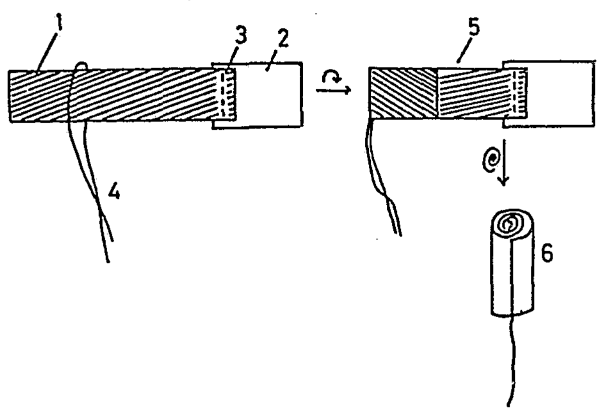


EP 0 163 179 A1

Croydon Printing Company Ltd.

Henkelstraße 67
4000 Düsseldorf, den 10. Mai 1984

HENKEL KGaA
ZR-FE/Patente
Dr. JG/Br

# P a t e n t a n m e l d u n g

D 7005 EP

## "Menstrualtampon"

Die Erfindung betrifft einen Menstrualtampon, bestehend aus einer äußeren Umhüllung aus Cellulosefasermaterial und einem Kern, der ganz oder teilweise aus saugfähigem, bakteriostatisch ausgerüsteten Fasermaterial besteht.

Es ist bekannt, daß in Menstrualtampons, die aus absorbierendem Fasermaterial bestehen und zur Aufnahmen von Körpersektreten, z.B. von Blut, Urin, Menstrualflüssigkeit dienen, bereits nach kurzer Zeit bakterielle Zersetzung des Sekrets durch ubiquitär vorhandene Bakterien und in Verbindung damit eine unangenehme Geruchsentwicklung erfolgt. Durch das Wachstum pathogener Keime werden darüber hinaus ernsthafte gesundheitliche Gefahren bedingt. Es hat nicht an Versuchen gefehlt, sanitäre Hygienemittel aus absorbierenden Fasermaterialien desodorierend und mikrobistatisch auszurüsten. Es wurden für diesen Zweck verschiedene Mikrobizide und Desodorantien vorgeschlagen, die zur Unterdrückung des Körpergeruchs geeignet sind.

Man hat auch schon vorgeschlagen, Kupferverbindungen zur Ausrüstung von Fasermaterialien für sanitäre Hygienemittel einzusetzen:

Aus DE-OS 31 35 410 ist z.B. ein absorbierender Körper aus Cellulose-Faservlies oder Watte bekannt, der mit der Lösung eines Kupfersalzes besprüht oder aus einer mit einer solchen Lösung behandelten Faser oder Watte angefertigt ist.

...

Die Verwendung mikrobizider Ausrüstungen bei Menstrualtampons hat vor allem den Nachteil, daß bei direktem Kontakt des mikrobiziden Stoffes oder des mikrobizid ausgerüsteten Fasermaterials mit der empfindlichen Vaginalschleimhaut unerwünschte Reizungen und vor allem eine schädliche Störung der natürlichen vaginalen Bakterienflora mit den daraus resultierenden Unzuträglichkeiten auftreten können.

Es wurde nun gefunden, daß diese Nachteile weitgehend vermieden werden, wenn ein Menstrualtampon aus einer äußeren Umhüllung aus Cellulosefasermaterial und einem Kern besteht, der ganz oder teilweise aus saugfähigem, bakteriostatisch ausgerüstetem Fasermaterial zusammengesetzt ist, so daß das bakteriostatisch ausgerüstete Fasermaterial bei Verwendung des Tampons selbst nicht mit der Vaginalschleimhaut in Berührung kommt. Bevorzugt sollte dabei die bakteriostatische Ausrüstung fest auf der Faser verankert sein, so daß sie durch die aufgenommenen Körpersekrete nicht von der Faser abgelöst wird und die mobilisierten Baktericide nicht an die Vaginalschleimhaut gelangen können. Eine solche Verankerung der bakteriostatischen Ausrüstung kann erfindungsgemäß in der Weise erfolgen, daß ein mit anionischen Gruppen modifiziertes und mit daran gebundenen, bakteriostatisch wirksamen Kationen ausgerüstetes Cellulosefasermaterial für den Kern des Tampons verwendet wird. Unter Cellulosefasermaterial wird vor allem Zellstoff, Baumwolle und Viskosefaser verstanden.

...

Anionische, salzbildende Gruppen lassen sich auf vielerlei Weise in das Cellulosemolekül einbauen. Solche anionisch modifizierte Cellulosefasern sind bekannt, einige davon auch im Handel erhältlich. Geeignet sind z.B. Cellulosefasern, die über den Sauerstoff an die Anhydroglukoseeinheiten gebundene Gruppen der allgemeinen Formel $-PO_3H^{(-)}$, $-(CH_2)_n-PO_3H^{(-)}$, $-(CH_2)_n-SO_3^{(-)}$ $(CH_2)_n-COO^{(-)}$ tragen, wobei n einen Wert von 1 - 3 haben kann. Bekannte Cellulosederivate dieser Art sind z.B. Cellulosephosphat, welches durch Veresterung von Cellulose mit Phosphorsäure zugänglich ist, Phosphonoethylcellulose, die durch Veretherung von Alkalicellulose mit Chlorethylphosphonat erhältlich ist, Phosphonomethylcellulose, die durch Veretherung von Cellulose mit Chlormethylphosphonat hergestellt werden kann, Sulfoethylcellulose, die durch Veretherung von Cellulose mit Chlorethansulfonat zugänglich ist und die analog herstellbare Sulfomethylcellulose und Sulfopropylcellulose. Durch Veretherung mit 1-Chlor-2-hydroxypropansulfonat ist die 1-Sulfo-2-hydroxypropylcellulose erhältlich.

Die Einführung von Carboxylgruppen in das Cellulosemolekül ist auf zwei prinzipiell verschiedenen Wegen möglich:

- durch physikalische Inkorporation von Carboxylgruppen tragenden Verbindungen in die Viskose, d.h. in eine als Cellulosexanthogenat gelöste Cellulose, unter Bildung sogenannter inkorporierter Viskosefasern (Alloy Fibers) oder

...

- durch chemische Umsetzung (Veretherung) der faserbildenden Cellulose mit Carboxylgruppen tragenden Reagentien unter Bildung von einheitlich mit z.B. Carboxyalkylgruppen der Formel $-(CH_2)_n-COOH$, in der n einen Wert von 1 - 3 haben kann, modifizierten Cellulosefasern.

Die physikalische Inkorporation Carboxylgruppen tragender Verbindungen in die Viskose wird z.B. durch Beimischen von Alkalisalzen von Acrylsäure-Homopolymerisaten, Acrylsäure-Methacrylsäure-Copolymerisaten, Methylvinylether-Maleinsäureanhydrid-Copolymerisaten, Alginsäure oder Carboxymethylcellulose zur Viskoselösung und anschließendes Verspinnen in üblicher Weise in ein Fällungsbad erreicht. Handelsübliche Fasern dieses Typs sind z.B. die ABSORBIT(R) - Fasern von Fa. Enka, die eine Mischfaser aus Viskose und Acrylsäure-Methacrylsäure-Copolymerisat darstellen. Solche Fasern sind nicht einheitlich modifiziert, sondern setzen sich aus modifizierten und unsubstituierten Faserfragmenten zusammen.

Einheitlich chemisch mit Carboxyalkylgruppen modifizierte Cellulosefasern sind für die Herstellung der bakteriostatisch ausgerüsteten Fasermaterialien besonders bevorzugt. In solchen Fasern ist die gesamte faserbildende Cellulose gleichmäßig modifiziert. Sie werden z.B. dadurch erhalten, daß man Cellulosefasern direkt nach Überführung in die Alkalicellulose mit z.B. Natriumchloracetat carboxymethyliert. Die so modifizierte

...

Cellulose kann über das Viskose-Spinnverfahren in ihrer Faserstruktur verbessert werden. Man kann aber auch eine über das Viskose-Spinnverfahren regenerierte Viskosefaser anschließend mit Chloressigsäure carboxymethylieren. Eine dritte Möglichkeit, zu einheitlich mit Carboxyalkylgruppen modifizierten Cellulosefasern zu gelangen, besteht darin, daß man während der Xanthogenierung der Viskoselösung z.B. Na-Chloracetat zusetzt und dann die carboxymethylierte Viskose wie üblich verspinnt. Solche einheitlich mit Carboxymethylgruppen modifizierte Viskosefasern sind im Handel erhältlich, z.B. unter der Bezeichnung VISCOSORB 1 N von Fa. Chemiefaser Lenzing.

Setzt man der Viskose-Lösung während der Xanthogenierung z.B. Acrylnitril zu, so erhält man nach Ablauf des Viskosespinnprozesses Viskosefasern, die aus niedrig substituierter Carboxyethylcellulose bestehen. Solche Fasern sind unter der Bezeichnung BAR-Fieber (Bondable Avisco Rayon) der FMC-Corporation im Handel.

Andere zur einheitlichen Modifizierung der Viskose durch Zusatz zur Viskoselösung während der Xanthogenierung geeignete Reagenzien sind z.B. Natrium-Vinylsulfonat, Na-Chlor-methansulfonat, Na-Chlormethanphosphonat. Man erhält auf diese Weise einheitlich chemisch modifizierte Viskosefasern mit Sulfoethylgruppen, Sulfomethylgruppen und Phosphonomethylgruppen.

Der Substitutionsgrad der für die Herstellung des bakteriostatisch ausgerüsteten Fasermaterials geeigneten Cellulosederivate sollte in Bezug auf die anionischen, salzbildenden Gruppen 0,01 bis 0,3 sein.

...

Zur Herstellung des bakteriostatisch ausgerüsteten Fasermaterials ist eine mit Carboxylgruppen, insbesondere mit Carboxymethylgruppen einheitlich chemisch modifizierte Cellulosefaser besonders bevorzugt, insbesondere ein Fasermaterial, welches aus einer durch das Viskose-Spinnverfahren regenerierten, mit Carboxymethylgruppen modifizierten Cellulose erhalten wird.

Als bakteriostatisch wirksame Kationen zur Ausrüstung des mit anionischen Gruppen modifizierten Cellulosefasermaterials kommen Schwermetallionen und Jonen von Übergangsmetallen wie Kupfer, Silber, Zink, Cadmium, Chrom- und Nickel sowie quartäre Ammoniumionen wie z.B. das Cetyltrimethylammoniumion oder das Lauryldimethylbenzylammoniumion in Frage. Besonders bevorzugt zur Ausrüstung der mit anionischen Gruppen modifizierten Cellulosematerialien sind Kupfer(II)Jonen.

Die für das bakteriostatisch auszurüstende Fasermaterial am besten geeigneten, mit Carboxymethylgruppen modifizierten Viskosefasern weisen einen Substitutionsgrad von 0,01 bis 0,3 auf, d.h. sie enthalten im Mittel etwa 0,01 bis 0,3 Carboxymethylgruppen pro Anhydroglukoseeinheit. Der Gehalt an gebundenem Kupfer sollte von 0,1 bis 2,0 Gewichtsprozent, bevorzugt von 0,2 bis 0,8 Gewichtsprozent des Fasermaterials ausmachen.

Die Herstellung des bakteriostatisch ausgerüsteten Fasermaterials ist aus bekannten, anionische, salzbildende Gruppen enthaltenden Fasern in einfacher Weise dadurch möglich, daß man

- die anionische, salzbildende Gruppen enthaltenden Fasern mit einer wäßrigen Lösung eines wasserlöslichen Salzes, z.B. eines Chlorids oder Sulfates des bakteriostatisch wirksamen Kations behandelt und

...

- die Fasern mit Wasser weitgehend salzfrei wäscht und trocknet.

Es ist außerdem vorteilhaft, wenn das bakteriostatisch ausgerüstete Fasermaterial ein hohes Wasserrückhaltevermögen aufweist. Bevorzugte Qualitäten weisen ein Wasserrückhaltevermögen von 80 % oder mehr gemessen nach DIN 53814 auf.

Das bevorzugt zu verwendende Fasermaterial mit hohem Wasserrückhaltevermögen läßt sich sehr leicht dadurch herstellen, daß man eine Carboxymethyl-Viskosefaser mit einem Substitutionsgrad von 0,01 bis 0,3 und mit entsprechend hohem Wasserrückhaltevermögen, z.B. die handelsüblichen Fasern Viscosorb ® 1S mit einem Wasserrückhaltevermögen von ca. 200 % und einem Substitutionsgrad von ca. 0,1 entweder in der Natriumsalzform oder nach Überführung in die freie Säureform in das beschriebene Verfahren einsetzt. Als wäßrige Kupfer-II-salzlösung eignet sich z.B. eine Lösung von 1 bis 20 g/l $CuSO_4 \cdot 5 H_2O$ in Wasser.

Die Behandlung erfolgt im allgemeinen ohne Wärmezufuhr über einen Zeitraum von wenigstens 1 Minute und ist in höchstens 1 Stunde beendet. Danach wird die Kupfersalzlösung, z.B. durch Abpressen, von der Faser abgetrennt, die Faser mit Wasser so lange gewaschen, bis das Waschwasser weitgehend frei von Sulfationen ist, das Wasser, z.B. durch Abpressen, von der Faser abgetrennt und die Faser im Luftstrom getrocknet. Es hat sich weiterhin als vorteilhaft erwiesen, wenn das bakteriostatisch ausgerüstete Fasermaterial einen Faser-pH-Wert, gemessen nach DIN 54275, von 4 bis 5 aufweist.

...

Ein solcher Faser-pH-Wert bewirkt, daß die erfindungsgemäßen Fasermaterialien einen gewissen Puffereffekt gegenüber aufgesaugten Körperflüssigkeiten haben und so den physiologisch günstigen, schwach sauren pH-Wert der Hautoberfläche herstellen, wodurch Reizungen und Anfälligkeit gegenüber Erkrankungen vermieden werden. Die Erzeugung eines mikrobiostatisch ausgerüsteten Fasermaterials mit einem Faser-pH-Wert von 4 bis 5 läßt sich nach diesem Verfahren leicht dadurch erreichen, daß man eine auf einen pH-Wert von 4 bis 5 eingestellte Kupfer-II-salzlösung einsetzt.

Das mit Cu-Jonen ausgerüstete Fasermaterial weist eine von der Menge des gebundenen Kupfers abhängige, mehr oder weniger ausgeprägte Blaufärbung auf, die unter Anwendungsbedingungen nicht auswaschbar ist. Die Farbe ist für die erfindungsgemäße Verwendung in sanitären Hygienemitteln nicht störend und entspricht durchaus hygienischen Vorstellungen.

Für die äußere Umhüllung der erfindungsgemäßen Tampons wird ein beliebiges, saugfähiges aber nicht antibakteriell ausgerüstetes Cellulosefasermaterial verwendet. Geeignet ist z.B. Zellstoffwatte oder Viskosewatte. Bevorzugt wird für die äußere Umhüllung ein pH-Wert regulierendes Cellulosefasermaterial eingesetzt. Solche Cellulosefasermaterialien sind z.B. mit Carboxymethylgruppen modifizierte Cellulosefasern, wie sie als Ausgangsstoffe für die Herstellung der bakteriostatisch ausgerüsteten Faser beschrieben wurden und die durch Säurebehandlung in die Säureform überführt wurden. Solche pH-Wert regulierende Cellulosefasern besitzen einen Faser-pH-Wert, gemessen nach DIN 54275 von weniger als 6, bevorzugt von 3,0 bis 4,0.

...

Die Herstellung der erfindungsgemäßen Menstrualtampons kann zum Beispiel nach den folgenden Verfahren erfolgen:

Ein Wattebandabschnitt aus Cellulosefasermaterial, z.B. aus Viskosewatte oder aus pH-Wert regulierender, carboxymethylierter Viskosewatte und ein Bandabschnitt eines bakteriostatisch ausgerüsteten Faservlieses werden so aneinandergefügt, daß eine zur Herstellung einer festen Verbindung ausreichende Überlappungszone von z.B. 2 bis 4 cm, entsteht. Die Länge der Bandabschnitte ist unter Berücksichtigung der Dicke der Bandabschnitte so zu bemessen, daß der Bandabschnitt des bakteriostatisch ausgerüsteten Faservlieses den Kern des Tampons und der Bandabschnitt des nicht bakteriostatisch ausgerüsteten Cellulosefasermaterials die äußere Hülle des Tampons bilden kann. Die Breite der beiden Bandabschnitte ist so bemessen, daß nach der bekannten Wickeltechnik und nach anschließendem Verpressen ein länglicher Menstrualtampon der gewünschten Größe hergestellt werden kann. Der Wattebandabschnitt aus nicht ausgerüstetem Cellulosematerial kann z.B. eine Länge von 6 bis 10 cm, der Bandabschnitt aus bakteriostatisch ausgerüstetem Faservlies eine Länge von 15 bis 25 cm haben. Der Wattebandabschnitt für die äußere Hülle kann z.B. eine Breite von 4 bis 6 cm, der bakteriostatisch ausgerüstete Faservlies-Bandabschnitt eine Breite von 3,5 bis 6 cm haben. Das Flächengewicht der Watte- und Vliesbänder kann, je nach der gewünschten Dicke des Tampons in einem Bereich von 100 bis 380 $g/m^2$ liegen. Die Längen- und Breitenangaben beziehen sich auf Tampons mittlerer Größe. Diese Abmessungen können natürlich der jeweils gewünschten Tampongröße angepasst werden. Der Bandabschnitt des

...

bakteriostatisch ausgerüsteten Faservlieses, der den Tamponkern bildet, darf aber nicht breiter sein als der nicht ausgerüstete Wattebandabschnitt, er sollte vielmehr bevorzugt um 10 bis 15 % schmaler als dieser sein.

In der Überlappungszone werden die beiden Bandabschnitte durch übliche Techniken, z.B. durch Vernadelung, Kalandrierung oder mit Hilfe von Preßluft fest miteinander verbunden. Das so vorbereitete Band wird dann, beginnend mit dem bakteriostatischen Faservlies zu einem Tampon in der Weise aufgewickelt, daß der nicht bakteriostatisch ausgerüstetete Wattebandabschnitt die äußere Umhüllung des Tampons bildet.

Eine weitere, technisch besondere einfache und kontinuierlich ausführbare Verfahrensvariante, die zu Menstrualtampons aus einer äußeren Umhüllung aus Cellulosefasermaterial und einem Kern aus teilweise bakteriostatisch ausgerüstetem Fasermaterial führt, wobei der Kern aus konzentrischen Lagen aus bakteriostatisch ausgerüstetem und nicht ausgerüstetem Fasermaterial besteht und äußere Umhüllung aus nicht ausgerüstetem Cellulosefasermaterial besteht, wird wie folgt ausgeführt:

Man legt auf ein Watteband aus Cellulosefasermaterial mittig Bandabschnitte eines bakteriostatisch ausgerüsteten Faservlieses auf und schneidet dann das Watteband in der Weise in gleich große Abschnitte, daß auf einer Schmalseite ein Überstand des Wattebandes über den Vliesbandabschnitt von 0,5 bis 2 cm resultiert. Die Länge und Breite der Bandabschnitte ist unter Berücksichtigung der Dicke so bemessen, daß nach der bekannten Wickeltechnik und nach anschließendem Verpressen ein länglicher Menstrualtampon der gewünschten Größe hergestellt werden kann. ...

Die Wattebandabschnitte können z.B. eine Länge von 20 bis 30 cm und eine Breite von 4 bis 6 cm haben. Die darauflliegenden bakteriostatisch ausgerüsteten Vliesbandabschnitte können z.B. eine Länge von 19 bis 29 cm und eine Breite von 3,5 bis 6 cm haben. Diese Angaben beziehen sich auf Watteband bzw. Vliesbandqualitäten mit einem Flächengewicht von 120 bis 380 $g/m^2$. Die Bandabschnitte des bakteriostatisch ausgerüsteten Faservlieses dürfen aber nicht breiter sein als das Watteband, sollten vielmehr bevorzugt um 10 bis 15 % schmaler sein als dieses. Die aufeinander liegenden Vlieslagen werden dann, beginnend mit dem Ende, an welchem kein Überstand des Wattebandes besteht, so aufgewickelt, daß das überstehende, nicht ausgerüstete Watteband die äußere Umhüllung des Tampons bildet. Nach dem Wickeln wird der Tampon in der üblichen Weise verpresst.

Die erfindungsgemäßen Tampons werden bevorzugt auch mit einer Rückholkordel ausgestattet. Vor dem Beginn des Wickelns wird diese Rückholkordel um das Ende des Wattebandabschnittes, der den Kern bilden soll, geknotet und das Ende des Bandabschnittes um die Rückholkordel umgeschlagen.

...

## Beispiele

1. Herstellung des für Beispiel 3 und 4 verwendeten bakteriostatisch ausgerüsteten Fasermaterials

Die Herstellung erfolgte ausgehend von einer handelsüblichen, carboxymethylierten Viskosefaser (Viscosorb® 1 S, Fa. Chemiefaser Lenzing) mit folgenden technischen Daten:

| | | |
|---|---|---|
| Wasserrückhaltevermögen | : | 180 - 200 ./. (DIN 53814) |
| Substitutionsgrad | : | 0,09 - 0,10 |
| Faser-pH-Wert | : | 7,0 - 7,5 (DIN 54275) |

1 kg Fasern der Type Viscosorb 1 S wurde mit 20 l einer Lösung von 20 g $CuSO_4 \cdot 5H_2O$ in 1000 ml Wasser, deren pH-Wert mit verdünnter Schwefelsäure auf 5 eingestellt war, 30 Minuten lang bei Raumtemperatur (20 °C) behandelt. Anschließend wurde das Fasermaterial auf eine Feuchte von 200 % abgepreßt und solange mit Wasser gewaschen, bis das Waschwasser sulfatfrei war. Danach wurde wiederum auf eine Feuchte von ca. 200 % abgepreßt und 4 Stunden im Umlufttrockenschrank bei 105 °C getrocknet. Das erhaltene Fasermaterial hatte folgende Daten:

| | | |
|---|---|---|
| Faser-pH-Wert | : | 5,6 (DIN 54275) |
| Kupfer-Gehalt | : | 1,45 Gew.-% |

2. Herstellung des für Beispiel 4 verwendeten (nicht bakteriostatisch ausgerüsteten), pH-Wert regulierenden Cellulosefasermaterials

Die Herstellung erfolgte ausgehend von der handelsüblichen carboxymethylierten Viskosefaser Viscosorb® 1 S

...

(Fa. Chemiefaser Lenzing), die in Beispiel 3 spezifiziert ist.

1 kg Fasern (Viscosorb® 1 S) wurden mit 20 l 0,2 %iger Salzsäure 30 Minuten lang bei Raumtemperatur behandelt. Anschließend wurde auf eine Feuchte, von 200 % abgepreßt und so lange mit vollentsalztem Wasser gewaschen, bis das Waschwasser neutral reagierte. Danach wurde wiederum auf eine Feuchte von ca. 200 % abgepreßt und 4 Stunden im Umlufttrockenschrank bei 105 °C getrocknet. Die Überprüfung des Faser-pH-Wertes nach dem Extrapolationsverfahren DIN 54275 erbrachte einen Wert von pH = 3,0.

3. Herstellung eines Menstrualtampons mit einem Kern aus bakteriostatisch ausgerüstetem Fasermaterial und einer nicht ausgerüsteten Hülle (Zeichnung I).

Auf einen Wattebandabschnitt aus neutraler Viskosefaser mit einem Flächengewicht von 165 $g/m^2$ von 5 cm Breite und 8 cm Länge (2) wird an einem Ende mittig überlappend (Überlappungszone 2,5 cm lang) ein schmaler Bandabschnitt eines gemäß Beispiel 1 bakteriostatisch ausgerüsteten Faservlieses von 4,5 cm Breite, 20 cm Länge und einem Flächengewicht von 232 $g/m^2$ (1) aufgelegt. Die beiden Bandabschnitte wurden in der Überlappungsstelle (3) durch Vernadelung verbunden. Um den bakteriostatisch ausgerüsteten Bandabschnitt wurde dann eine Rückholkordel (4) geknotet und nach Teilumschlag (5) des bakteriostatisch ausgerüsteten Wattebandes über die Kordel ein Wickel in der Weise gebildet, daß der nicht ausgerüstete Wattebandabschnitt die äußere Umhüllung bildete. Die Verpressung zum fertigen Tampon erfolgte nach dem für Wickeltampons üblichen Verfahren.

...

Es wurde ein Wickeltampon erhalten, dessen äußere Umhüllung vollständig aus nicht bakteriostatisch ausgerüsteter Viskose-Watte besteht. Der Kern besteht hingegen ganz aus bakteriostatisch ausgerüstetem Fasermaterial.

4. Herstellung eines Menstrualtampons mit einem Kern aus konzentrischen Lagen bakteriostatisch ausgerüsteten und nicht ausgerüsteten Fasermaterials und einer nicht bakteriostatisch ausgerüsteten Hülle (Zeichnung II).

Auf ein Watteband aus pH-Wert regulierendem Cellulosefasermaterial gemäß Beispiel 2 mit einem Flächengewicht von 165 g/$m^2$, von 5 cm Breite (8) wurden taktweise Bandabschnitte von 4 cm Breite und 23 cm Länge eines gemäß Beispiel 1 bakteriostatisch ausgerüsteten Faservlieses (7) mit einem Flächengewicht von 190 g/$m^2$ mittig im Abstand von 1 cm voneinander aufgelegt. Das Watteband wurde dann zu 24 cm langen Abschnitten so geschnitten, daß es jeweils an einer Seite um 1 cm über den ausgerüsteten Vliesbandabschnitt überstand. Nach Umknoten der Rückholkordel (14) und Teileinschlag der Seite der Vlieslagen, die keinen Überstand aufwies,über die Kordel, wobei die ausgerüstete Vlieslage nach innen gelangte (15), wurde, beginnend mit dem eingeschlagenen Ende,ein Wickel (16) in der Weise gebildet, daß der nicht ausgerüstete, überstehende Wattebandabschnitt die äußere Umhüllung bildete. Die Verpressung zum fertigen Tampon (17) erfolgte nach dem für Wickeltampons üblichen Verfahren.

Es wurde ein Wickeltampon erhalten, dessen äußere Umhüllung vollständig aus pH-Wert regulierender, nicht bakteriostatisch ausgerüsteter Watte besteht. Der Kern

...

besteht hingegen aus konzentrischen Lagen bakteriostatisch ausgerüsteten und nicht ausgerüsteten, pH-Wert regulierenden Fasermaterialien.

Die Beispiele 3 und 4 werden durch die Abbildungen I und II näher erläutert.

Abbildung I

1. bakteriostatisch ausgerüsteter Wattebandabschnitt
2. Wattebandabschnitt aus neutraler Viskosewatte
3. Überlappungsbereich zur Vernadelung
4. Rückholkordel
5. Teileinschlag über die Rückholkordel
6. Loser Wickeltampon vor dem Verpressen

Abbildung II

7. bakteriostatisch ausgerüstetes Faservlies
8. Watteband aus pH-Wert regulierender Viskose
9. Transportband
10. Leitband
11. Schneideeinheit
12. aufeinanderliegende Wattebandabschnitte
13. Tamponmaschine zum Wickeln und Pressen
14. Rückholkordel
15. Teileinschlag über die Rückholkordel
16. Loser Wickeltampon vor dem Verpressen
17. fertiger gepreßter Menstrualtampon

...

## Patentansprüche

1. Menstrualtampon, bestehend aus einer äußeren Umhüllung aus Cellulosefasermaterial und einem Kern, dadurch gekennzeichnet, daß der Kern ganz oder teilweise aus saugfähigem, bakteriostatisch ausgerüstetem Fasermaterial besteht.

2. Menstrualtampon nach Anspruch 1, dadurch gekennzeichnet, daß der Kern ganz oder teilweise aus einem mit anionischen Gruppen modifizierten und mit daran gebundenen bakteriostatisch wirksamen Kationen ausgerüstetem Cellulosefasermaterial besteht.

3. Menstrualtampon nach Anspruch 2, dadurch gekennzeichnet, daß die anionischen Gruppen über den Sauerstoff an die Anhydroglucoseeinheiten gebundenen Gruppen der Formeln

$-PO_3H^{\ominus}$, $-(CH_2)_2-PO_3H^{\ominus}$, $-(CH_2)_2-SO_3^{\ominus}$ oder $-(CH_2)_2-COO^{\ominus}$

sind, in welchen n einen Wert von 1 bis 3 hat, der Substitutionsgrad der Cellulosederivate 0,01 bis 0,3 ist und die bakteriostatisch wirksamen Kationen Kupfer II Ionen sind.

4. Menstrualtampon nach Anspruch 1 bis 3, dadurch gekennzeichnet, daß das bakteriostatisch ausgerüstete Fasermaterial aus einer einheitlichen, mit Carboxymethylgruppen modifizierten Viskosefaser besteht und mit Kupferionen in einer Menge von 0,01 bis 2,0 Gewichtsprozent, bevorzugt 0,2 bis 0,8 Gewichtsprozent des Fasermaterials ausgerüstet ist.

...

5. Menstrualtampon nach Anspruch 1 bis 4, dadurch gekennzeichnet, daß die äußere Umhüllung aus Cellulosewatte oder aus mit Carboxymethylgruppen modifizierter Viskosewatte besteht.

6. Verfahren zur Herstellung von Menstrualtampons aus einer äußeren Umhüllung aus Cellulosefasermaterial und einem Kern aus bakteriostatisch ausgerüstetem Fasermaterial, dadurch gekennzeichnet, daß man
- einen Wattebandabschnitt aus Cellulosefasermaterial und einen Bandabschnitt eines Bakteriostatisch ausgerüsteten Faservlieses so aneinanderfügt, daß eine zur festen Verbindung ausreichende Überlappungszone entsteht, die beiden Bandabschnitte in der Überlappungszone verbindet und beginnend mit dem bakteriostatisch ausgerüsteten Faservlies zu einem Tampon in der Weise aufwickelt und verpreßt, daß der Wattebandabschnitt die Umhüllung des Tampons bildet.

7. Verfahren zur Herstellung von Menstrualtampons aus einer äußeren Umhüllung aus Cellulosefasermaterial und einem Kern aus teilweise bakteriostatisch ausgerüstetem Fasermaterial, dadurch gekennzeichnet, daß man
- auf ein Watteband aus Cellulosefasermaterial Bandabschnitte eines bakteriostatisch ausgerüsteten Faservlieses auflegt, das Watteband so schneidet, daß wenigstens auf einer Schmalseite ein Überstand des Wattebandes über das Faservlies von 0,5 bis 2 cm resultiert und die aufeinanderliegenden Vlieslagen zu einem Tampon in der Weise aufwickelt und verpreßt, daß das Watteband die Umhüllung des Tampons bildet.

...

8. Verfahren nach Anspruch 6 oder 7, dadurch gekennzeichnet, daß der Bandabschnitt des bakteriostatisch ausgerüsteten Faservlieses um 0,5 bis 2 cm schmaler ist als der Wattebandabschnitt.

D 7005 EP

1/2

0163179

Henkel KGaA
ZR-FE/Patente

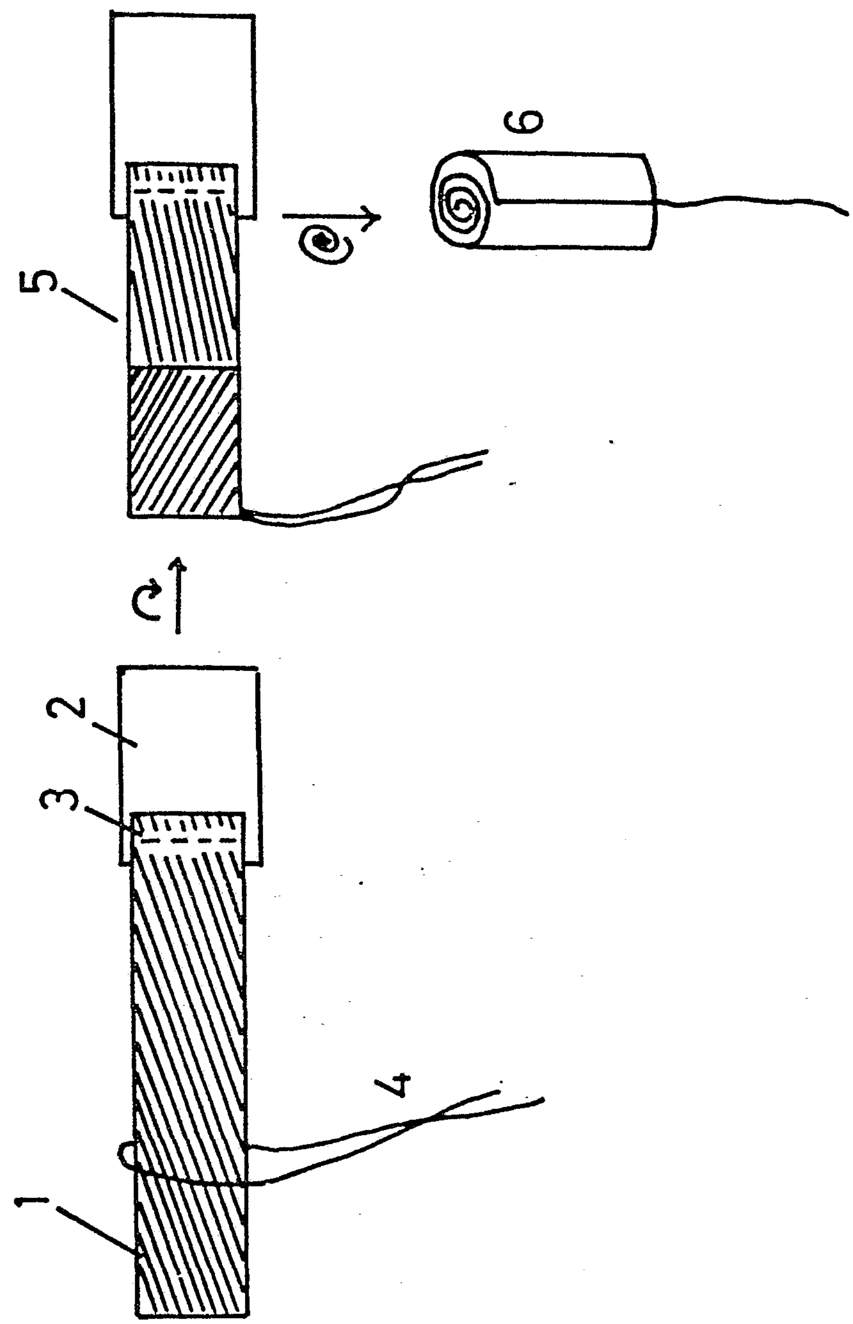


Abb.I

Henkel KGaA
ZR-FE/Patente

Abb. II

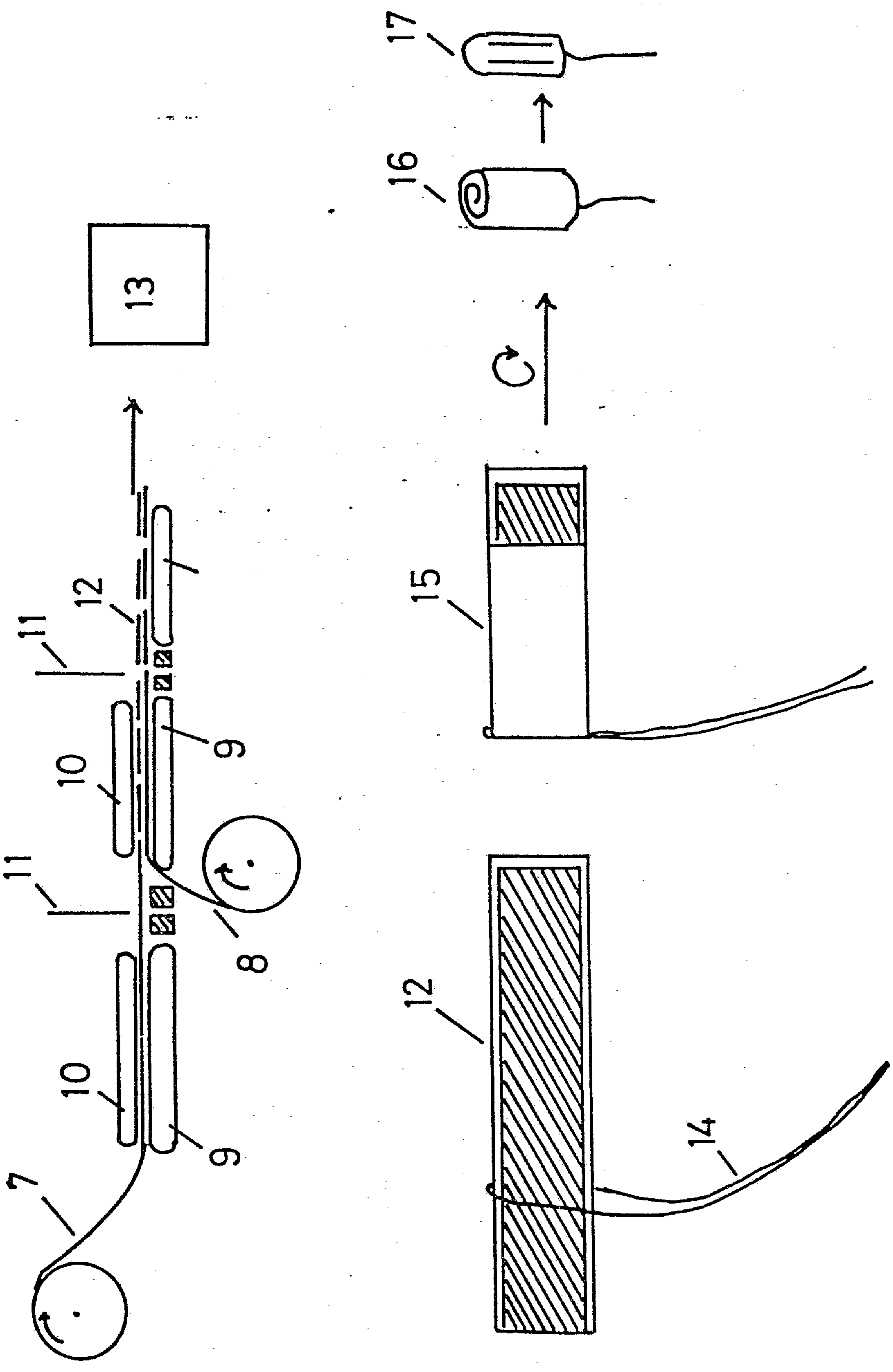

Europäisches Patentamt

**0163179**
Nummer der Anmeldung

## EUROPÄISCHER RECHERCHENBERICHT

EP 85 10 5736

| EINSCHLÄGIGE DOKUMENTE | | | |
| --- | --- | --- | --- |
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | FR-A-1 540 077 (THE CALICO PRINTERS ASSOC.) * Zusammenfassung * --- | 1-8 | A 61 L 15/00<br>A 01 N 25/10<br>A 01 N 59/20 |
| X | FR-A-1 499 358 (MOSKOVSKY TEKSTILNY INSTITUT) * Beispiel 2 * --- | 1,2 | |
| X | FR-A-1 499 788 (MOSKOVSKY TEXTILNY INSTITUT) * Beispiel 3 * --- | 1-3 | |
| A | US-A-2 856 330 (H.N.VAGINIUS) * Ansprüche 1-4 * --- | 1 | |
| D,A | GB-A-2 083 748 (LANDSTINGENS INKÖPSCENTRAL) * Ansprüche 1,6 * ----- | 1 | RECHERCHIERTE SACHGEBIETE (Int. Cl.4)<br>A 61 L<br>A 01 N<br>D 06 M |

Der vorliegende Recherchenbericht wurde für alle Patentansprüche erstellt.

| Recherchenort | Abschlußdatum der Recherche | Prüfer |
| --- | --- | --- |
| DEN HAAG | 25-06-1985 | PELTRE CHR. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82